# EUROPÄISCHE PATENTSCHRIFT

(11) **EP 0 717 632 B1**
(45) Veröffentlichungstag und Bekanntmachung des Hinweises auf die Patenterteilung: **17.10.2001**
(21) Anmeldenummer: 95917891.4
(22) Anmeldetag: 09.05.1995
(51) Int. Cl.: A61K 35/78

(54) **IMMUNSTÄRKENDES NAHRUNGSERGÄNZUNGSMITTEL**
A FOOD ADDITIVE FOR IMPROVING IMMUNITY
COMPLEMENT ALIMENTAIRE AMELIORANT LES DEFENSES IMMUNITAIRES

(30) Priorität: 09.05.1994 DE 4416402
(43) Veröffentlichungstag der Anmeldung: 26.06.1996
(73) Patentinhaber: Carl, Helmut, 91282 Betzenstein (DE)
(72) Erfinder: CARSTENS, Harro, D-91186 Büchenbach (DE); CARL, Helmut, D-91282 Betzenstein (DE)
(74) Vertreter: Fritsche, Rainer, Dipl.-Wirtsch.-Ing.
(86) Internationale Anmeldenummer: DE9500605
(87) Internationale Veröffentlichungsnummer: WO9605848

(56) Entgegenhaltungen:
- DATABASE WPI Section Ch, Week 9407 Derwent Publications Ltd., London, GB; Class B04, AN 94-054731 & RU,C,2 000 793 ( MOSHANOVA N S) , 15.Oktober 1993
- DATABASE WPI Week 199201, Derwent Publications Ltd., London, GB; AN 1992-002621 & JP 3 255 031 A (TAIYO PERFUMERY CO LTD) 13 November 1991

## Beschreibung

Die Erfindung betrifft ein immunstärkendes Nahrungsergänzungsmittel.

Das Immunsystem stellt in seiner Gesamtheit das körpereigene Schutzsystem des Menschen und der höheren Wirbeltiere zur Abwehr von körperfremdem Material dar. Nach der im "Lexikon der Biochemie und Molekularbiologie" (Herder-Verlag, Freiburg, Basel, Wien 1991) gegebenen Definition umfaßt es die Gesamtheit aller Immunzellen eines Organismus einschließlich aller immunologisch kompetenten Organe wie Lymphknoten, Milz, Thymus, gastrointestinales Lymphgewebe, Knochenmark und humoralen Komponenten (Antikörper). Die Aufgabe des Immunsystems besteht neben dem Erkennen von körperfremden Material auch in der Einleitung von Abwehrreaktionen und darüber hinaus im Etablieren eines körpereigenen "immunologischen Gedächtnisses", in bezug auf spezifische Antigene. Ein Teil dieser Aufgabe betrifft die immunologische Überwachung von Entartungsvorgängen bei körpereigenen Zellen, wie sie z. B. bei Krebs und in der Endphase einer HIV-Infektion (Aids) auftreten. Es gibt Hinweise darauf, daß eine allgemeine Schwächung des Immunsystems zu einer erhöhten Fehlerhäufigkeit bei der Ausführung dieser Überwachungsfunktion führt. Werden im Zuge dieser Entwicklung nicht alle transformierten Zellen eliminiert, kann es zur Ausbildung von Tumoren kommen.

In Teilbereichen der Medizin waren in der jüngsten Vergangenheit Entwicklungen zu verzeichnen, bei denen nicht-konventionelle Behandlungsverfahren ergänzend oder alternativ an die Seite der etablierten Medizin getreten sind, die Grenzen zwischen etablierten und nicht-konventionellen Behandlungsverfahren also aufgelöst wurden. Ein Aspekt dieser Entwicklung ist der zunehmende Stellenwert einer Ganzheitsbehandlung von Körper, Seele und Geist, wie es sich beispielsweise in der Integration fernöstlicher Denkansätze manifestiert, die die Ursache von Krankheiten auf gestörte Harmoniemuster zurückführen. Gefördert wurde diese Entwicklung auch durch eine zunehmend kritische Einschätzung und Neubewertung des Stellenwerts der Schulmedizin, beispielsweise im Zusammenhang auf eine kritische Analyse der Nebenwirkungen von klassischen Medikamenten. Dies führte zur Entwicklung von therapeutischen Strategien, bei denen die auf solchen Medikamenten beruhende Schulmedizin nur ein Teilsegment darstellt und die biologische Zusatzbehandlung - beispielsweise aus dem Bereich der Naturheilkunde - eine wachsende Bedeutung gewinnt.

Trotz verschiedener Erfolge der Naturheilkunde wie beispielsweise in der Hydrotherapie (Kneipp) fehlt ein Ansatz, mit Kräftigungs- und Stärkungsmitteln vorbeugend und im Sinne einer Langzeitbehandlung stabilisierend auf das Immunsystem einzuwirken.

Die Erfindung stellt sich die Aufgabe, ein immunstärkendes Nahrungsergänzungsmittel auf pflanzlicher Basis zur Verfügung zu stellen.

Diese Aufgabe wird gelöst mit einem immunstärkenden Nahrungsergänzungsmittel gemäß Anspruch 1. Weiterbildungen und bevorzugte Ausführungsformen sind in den Unteransprüchen beschrieben.

Der Erfindung liegt der Gedanke zugrunde, die positiven Wirkungen bekannter Heilkräuter mit dem Eugenolgehalt von Pflanzenölen zu kombinieren, wodurch sich über einen synergistischen Effekt eine stärkende Wirkung auf das menschliche Immunsystem ergibt. Dieser synergistische Effekt wird unterstützt durch Heilkräuter mit über den Magen/Darm-Trakt entgiftender Wirkung. Durch die Kombination dieser Heilkräuter mit eugenolhaltigen Pflanzenölen ergibt sich eine Verstärkung ihrer antibakteriellen, antiviralen und antimycotischen Wirkung. Heilkräuter mit über den Magen/Darm-Trakt entgiftender Wirkung sind deshalb besonders bevorzugt.

Heilkräuter (Heilpflanzen, Arzneimittelpflanzen) sind Pflanzen, die aufgrund ihrer Inhaltsstoffe in der Heilpflanzenkunde (Phytotherapie) verwendet werden. Heilpflanzen nehmen traditionell in der Volksmedizin einen wichtigen Platz ein, jedoch stellt die Heilpflanzenkunde mittlerweile eine wissenschaftlich begründete Heilmethode dar, die sich im Laufe der Jahrhunderte aus der Kräuterheilkunde entwickelt hat. Wirkstoffe der Heilpflanzen bzw. Heilkräuter sind in erster Linie Alkaloide, Glykoside, etherische Öle und die Gruppe der Gerb- und Bitterstoffe. Auch heute noch sind Heilpflanzen der Ausgangspunkt zur Herstellung von ca. 55 % aller Arzneimittel. Es ist unbestritten, daß der bei weitem nicht vollständig erfaßte Bestand an Pflanzen mit pharmakologisch wirksamen Inhaltsstoffen in den tropischen Wäldern einen der wahren Schätze der Menschheit darstellt.

Erfindungsgemäß wird ein alkoholischer (ethanolischer) Heilkräuterextrakt verwendet. Dieser kann zu etwa 20 Gew-% aus Ethanol und zum Rest aus extrahierten Festsubstanzen bestehen.

Erfindungsgemäß werden die alkoholischen (ethanolischen) Heilkräuterextrakte mit eugenolhaltigen Pflanzenölen gemäß den Ansprüchen kombiniert. Der Begriff "Pflanzenöl" stellt dabei eine Sammelbezeichnung für etherische Öle bzw. Essenzen dar, die durch physikalische Verfahren aus Pflanzen oder Pflanzenteilen gewonnen werden können. Im allgemeinen handelt es sich dabei um destillative Verfahren, beispielsweise um die Wasserdampfdestillation. Dabei werden erntefrische Pflanzenteile entweder direkt oder nach Anrühren mit Wasser durch Wasserdampfdestillation ausgezogen. Beim Anrühren mit Wasser (Einmaischen) kann es in gewissem Umfang zu Gärungsprozessen kommen, bei denen in bestimmtem Umfang Abbaureaktionen ablaufen. Nach der Kondensation der Dämpfe wird das Destillat aufgefangen und das so gewonnene etherische Öl gegebenenfalls durch nochmalige Destillation mit Wasserdampf rektifiziert.

Erfindungsgemäß werden eugenolhaltige Pflanzenöle bzw. etherische Öle eingesetzt. Eugenol (4-Allyl-2-methoxyphenol, C₁₂H₁₂O₂) ist Bestandteil zahlreicher etherischer Öle und beispielsweise in Nelkenöl mit 80 %, in Pimentbeeren-Öl mit 60 bis 90 % und in Bayöl mit 60 % enthalten.

Neben den alkoholischen (ethanolischen) Heilkräuterextrakten und den eugenolhaltigen Pflanzenölen enthält ein erfindungsgemäßes immunstärkendes Nahrungsergänzungsmittel noch gegebenenfalls übliche Stabilisierungsmittel und Zusatzstoffe.

Bevorzugt werden Extrakte von Heilkräutern verwendet, die über den Magen/Darm-Trakt entgiften. Die Entgiftung des Magen/Darm-Traktes hat dabei eine das Immunsystem unterstützende Funktion. Eine Entgiftung wird durch Verbesserung der Muskelspannung und damit der Elastizität der Magen- und Darmwände erreicht. Damit wird der Darmfluß beschleunigt, und Giftstoffe haben keine Gelegenheit mehr, längere Zeit durch die Darmwände schädigend auf den Organismus einzuwirken.

Bevorzugte erfindungsgemäße Heilkräuterextrakte sind Extrakte aus Aloe, Myrrhe, Saflor, Sennesblätter, Manna, Eberwurz, Angelikawurzel, Kampfer, Zittwerwurzel, Theriak, Brennessel, Ringelblume, Labkraut, Sauerklee, Kalmus, Schöllkraut, Wegmalve, Weidenröschen, Schafgarbe, Mistel, Zinnkraut, Rosmarin, Salbei und Majoran.

Diese Heilkräuter sind Beispiele für solche mit den Magen/Darm-Trakt entgiftender Wirkung.

Bevorzugte erfindungsgemäße eugenolhaltige Pflanzenöle sind Nelken-Öl, Pimentbeeren-Öl, Bay-Öl, Lorbeerblätter-Öl, Basilikum-Öl, Eukalyptus-Öl und Citronella-Öl.

Die vorstehend genannten, besonders bevorzugten Heilkräuterextrakte (H) bzw. eugenolhaltigen etherischen Öle (E) sind in der nachfolgenden Tabelle I zusammengestellt.

**Tabelle I**

| Heilkräuterextrakte (H) | | |
|---|---|---|
| Abk. | Name | lat. Bezeichnung |
| H 1 | Aloe | aloe ferox |
| H 2 | Myrrhe | myrrha |
| H 3 | Saflor | flores carthami |
| H 4 | Sennesblätter | folia sennae |
| H 5 | Manna | manna |
| H 6 | Eberwurz | radix carlinae |
| H 7 | Angelikawurzel | radix angelicae |
| H 8 | Kampfer | camphora |
| H 9 | Zittwerwurzel | rhizoma zedoariae |
| H10 | Theriak | electuarium theriaca |
| H11 | Brennessel | urtica dioica |
| H12 | Ringelblume | calendula officinalis |
| H13 | Labkraut | galium verum |
| H14 | Sauerklee | oxalis acetosella |
| H15 | Kalmus | acorus calanus |
| H16 | Schöllkraut | chelidonium majus |
| H17 | Wegmalve | malva vulgaris |
| H18 | Weidenröschen | epilosbium roseum |
| H19 | Schafgarbe | achillea milleflorum |
| H20 | Mistel | viscum alba |
| H21 | Zinnkraut | equisetum arvense |
| H22 | Rosmarin | rosmarinus officinalis |
| H23 | Salbei | salvia officinalis |
| H24 | Majoran | origanum majorana |

| Eugenolhaltige etherische Öle (E) | | |
|---|---|---|
| Abk. | Name | lat. Bezeichnung |
| E 1 | Nelken-Öl | eugenia caryophyllata |
| E 2 | Pimentbeeren-Öl | pimento officinalis |
| E 3 | Bay-Öl | pimento racemosa |
| E 4 | Lorbeerblätter-Öl | laurus nobilis |
| E 5 | Basilikum-Öl | ocimunm basilicum |
| E 6 | Eukalyptus-Öl | eucalyptus globulus |
| E 7 | Citronella-Öl | cymbopogon winterianus |

Die Heilkräuterextrakte H1 bis H24 bzw. eugenolhaltigen etherischen Öle E1 bis E7 können in beliebiger Weise miteinander kombiniert werden, besonders bevorzugt sind jedoch sechs Ausführungsformen der Erfindung, die sich aus der nachstehenden Tabelle II ergeben. Dabei sind die jeweils eingesetzten Komponenten H bzw. E mit einem "x" bezeichnet.

**Tabelle II**

| Heilkräuterextrakte (H) | | | Ausführungsform | | | | | |
|---|---|---|---|---|---|---|---|---|
| | | | 1 | 2 | 3 | 4 | 5 | 6 |
| Abk. | Name | lat. Bezeichnung | enthalten (x) | | | | | |
| H 1 | Aloe | aloe ferox | x | x | x | x | x | x |
| H 2 | Myrrhe | myrrha | x | x | x | x | x | x |
| H 3 | Saflor | flores carthami | x | x | x | x | x | x |
| H 4 | Sennesblätter | folia sennae | x | x | x | x | x | x |
| H 5 | Manna | manna | x | x | x | x | x | x |
| H 6 | Eberwurz | radix carlinae | x | x | x | x | x | x |
| H 7 | Angelikawurzel | radix angelicae | x | x | x | x | x | x |
| H 8 | Kampfer | camphora | x | x | x | x | x | x |
| H 9 | Zittwerwurzel | rhizoma zedoariae | x | x | x | x | x | x |
| H10 | Theriak | electuarium theriaca | x | x | x | x | x | x |
| H11 | Brennessel | urtica dioica | x | x | | | x | |
| H12 | Ringelblume | calendula officinali | x | x | | x | | |
| H13 | Labkraut | galium verum | x | | x | x | | |
| H14 | Sauerklee | oxalis acetosella | x | | | | | |
| H15 | Kalmus | acorus calanus | x | x | x | | | |
| H16 | Schöllkraut | chelidonium majus | x | x | x | x | | |
| H17 | Wegmalve | malva vulgaris | x | x | | x | | |
| H18 | Weidenröschen | epilosbium roseum | x | | x | | | |
| H19 | Schafgarbe | achillea milleflorum | x | x | | | | |
| H20 | Mistel | viscum alba | x | | x | x | x | |
| H21 | Zinnkraut | equisetum arvense | x | | x | | | |
| H22 | Rosmarin | rosmarinus officinalis | x | | | | | |
| H23 | Salbei | salvia officinalis | x | | | | | |
| H24 | Majoran | origanum majorana | x | | | | | |

| Eugenolhaltige Etherische Öle (E) | | | | | | | | |
|---|---|---|---|---|---|---|---|---|
| Abk. | Name | lat. Bezeichnung | | | | | | |
| E 1 | Nelken-Öl | eugenia caryophyllata | x | x | x | x | x | x |
| E 2 | Pimentbeeren-Öl | pimento officinalis | x | | | | | |
| E 3 | Bay-Öl | pimento racemosa | x | x | | | | |
| E 4 | Lorbeerblätter-Öl | laurus nobilis | x | x | x | x | | |
| E 5 | Basilikum-Öl | ocimunm basilicum | x | x | | | | |
| E 6 | Eukalyptus-Öl | eucalyptus globulus | x | | x | | | |
| E 7 | Citronella-Öl | cymbopogon winterianus | x | x | x | | | |

Die vorstehend aufgeführten Ausführungsformen sind in gleicher Weise geeignet, wobei jedoch die Magenverträglichkeit in der Reihenfolge 1 bis 6 zunimmt. Bei Magenempfindlichkeit sollte z. B. Ausführungsform 1 reichlich verdünnt mit Tee oder Fruchtsaft eingenommen werden.

Bevorzugt enthält das immunstabilisierende Nahrungsergänzungsmittel 40 bis 60 Gewichtsteile Heilkräuterextrakte auf 60 bis 40 Gewichtsteile Pflanzenöle, besonders bevorzugt 45 bis 55 Gewichtsteile Heilkräuterextrakte auf 55 bis 45 Gewichtsteile Pflanzenöle. Aufgrund des Ethanolgehalts der Heilkräuterextrakte enthält ein solches Gemisch 8 bis 12 Gew-% Ethanol bezogen auf die Summe aus Heilkräuter-Inhaltsstoffen, Pflanzenölen und Ethanol, der Rest (92 bis 88 Gew-%) entfällt auf die Summe aus Heilkräuter-Inhaltsstoffen und Pflanzenölen.

Bevorzugt sind die einzelnen Heilkräuterextrakte zueinander in im wesentlichen gleicher Menge vorhanden, wobei jedoch wahlweise Aloe gegenüber dieser Menge erhöht und gleichzeitig Saflor so erniedrigt sein kann, daß sich die Erhöhung und die Erniedrigung ausgleichen.

Die etherischen Öle sind zueinander so aufgeteilt, daß jedes Öl ohne Berücksichtigung von Nelken-Öl mit 10 bis 12 Gew-% vorhanden ist und Nelken-Öl den Rest ausmacht.

Die galenische Herstellung des immunstärkenden Nahrungsergänzungsmittels erfolgt durch Mischen der ethanolischen Heilkräuterextrakte und der eugenolhaltigen Pflanzenöle im jeweils beabsichtigten Verhältnis. Es bilden sich zwei Phasen, die durch Schütteln homogenisiert werden können, wobei jedoch bei längerem Stehen wieder Entmischung eintritt. Eine homogene, dispergierte Phase kann durch Zusatz eines Emulgators (z. B. Lecithin) stabilisiert werden. Im allgemeinen wird auf einen Emulgator jedoch verzichtet. In diesem Fall sollte das Nahrungsergänzungsmittel vor der Einnahme durch Schütteln homogenisiert werden.

Als Dosierung für das erfindungsgemäße immunstärkende Nahrungsergänzungsmittel empfiehlt sich ein Gabe von drei mal zehn Tropfen pro Tag, die mit Flüssigkeit (beispielsweise warmer Tee oder Fruchtsaft) verdünnt werden können.

## Patentansprüche

1. Immunstärkendes Nahrungsergänzungsmittel, entaltend einen oder mehrere ethanolische Heilkräuterextrakte, ein oder mehrere eugenolhaltige Pflanzen-öle sowie gegebenenfalls übliche Stabilisierungsmittel und Zusatzstoffe enthält,
**gekennzeichnet durch**
(a) 40 bis 60 Gewichtsteile der Heilkräuterextrakte von Aloe, Myrrhe, Saflor, Sennesblätter, Manna, Eberwurz, Angelikawurzel, Kampfer, Zittwerwurzel und Theriak und
(b) 60 bis 40 Gewichtsteile des Pflanzenöls Nelken-Öl, wobei
(c) die Heilkräuterextrakte untereinander in im wesentlichen gleicher Menge vorhanden sind.

2. Nahrungsergänzungsmittel nach Anspruch 1, **dadurch gekennzeichnet, daß** zusätzlich die Heilkräuterextrakte von Brennessel und Mistel in im wesentlichen gleicher Menge wie die anderen Heilkräuterextrakte vorhanden sind.

3. Nahrungsergänzungsmittel nach Anspruch 1 oder 2, **dadurch gekennzeichnet daß** zusätzlich die Heilkräuterextrakte von Ringelblume, Labkraut, Schöllkraut, Wegmalve und Mistel in im wesentlichen gleicher Menge wie die anderen Heilkräuterextrakte vorhanden sind und zusätzlich das Pflanzenöl Lorbeerblätter-Öl vorhanden ist, wobei die Menge des zusätzlichen Pflanzen-Öls 10 bis 12 Gew.-% der Menge des Nelken-Öls beträgt.

4. Nahrungsergänzungsmittel nach Anspruch 1, **dadurch gekennzeichnet, daß** zusätzlich die Heilkräuterextrakte von Labkraut, Kalmus, Schöllkraut, Weidenröschen, Mistel und Zinnkraut in im wesentlichen gleicher Menge wie die anderen Heilkräuterextrakte vorhanden sind und zusätzlich die Pflanzen-öle Lorbeerblätter-Öl, Eukalyptus-Öl und Citronella-Öl vorhanden sind, wobei die Menge der zusätzlichen Pflanzen-Öle jeweils 10 bis 12 Gew.-% der Menge des Nelken-Öls beträgt.

5. Nahrungsergänzungsmittel nach Anspruch 1, **dadurch gekennzeichnet, daß** zusätzlich die Heilkräuterextrakte von Brennessel, Ringelblume, Kalmus, Schöllkraut, Wegmalve und Schafgarbe in im wesentlichen gleicher Menge wie die anderen Heilkräuterextrakte vorhanden sind und zusätzlich die Pflanzenöle Bay-Öl, Lorbeerblätter-ÖI, Basilikum-Öl und Citronella-Öl vorhanden sind, wobei die Menge der zusätzlichen Pflanzen-Öle jeweils 10 bis 12 Gew.-% der Menge des Nelken-Öls beträgt.

6. Nahrungsergänzungsmittel nach Anspruch 1, **dadurch gekennzeichnet, daß** zusätzlich die Heilkräuterextrakte von Brennessel, Ringelblume, Labkraut, Sauerklee, Kalmus, Schöllkraut, Wegmalve, Weidenröschen, Schafgarbe, Mistel, Zinnkraut, Rosmarin, Salbei und Majoran in im wesentlichen gleicher Menge wie die anderen Heilkräuterextrakte vorhanden sind und zusätzlich die Pflanzenöle Pimentbeeren-Öl, Bay-Öl, Lorbeerblätter-Öl, Basilikum-Öl, Eukalyptus-Öl und Citronella-Öl vorhanden sind, wobei die Menge der zusätzlichen Pflanzen-Öle jeweils 10 bis 12 Gew.-% der Menge des Nelken-Öls beträgt.

7. Nahrungsergänzungsmittel nach einem der vorhergehenden Ansprüche, **dadurch gekennzeichnet, daß** es bezogen auf die Summe aus Heilkräuter-lnhaltsstoffen, Pflanzenölen und Ethanol 8 bis 12 Gew-% Ethanol und 92 bis 88 Gew-% Heilkräuter-Inhaltsstoffe plus Pflanzenöle enthält.

8. Nahrungsergänzungsmittel nach einem der vorhergehenden Ansprüche, **dadurch gekennzeichnet, daß** es einen Emulgator enthält.

9. Nahrungsergänzungsmittel nach Anspruch 9, **dadurch gekennzeichnet, daß** der Emulgator Lecithin ist.

10. Nahrungsergänzungsmittel nach einem der Ansprüche 1 bis 9, **dadurch gekennzeichnet, daß** Aloe gegenüber der Menge gemäß Merkmal (c) erhöht und gleichzeitig Saflor so erniedrigt ist, daß sich die Erhöhung und die Erniedrigung ausgleichen.

11. Nahrungsergänzungsmittel nach einem der Ansprüche 2 bis 10, **gekennzeichnet durch** 45 bis 55 Gewichtsteile Heilkräuterextrakte auf 55 bis 45 Gewichtsteile Pflanzenöl(e).

## Claims

1. An immune-strengthening food supplement containing one or more ethanolic medicinal herb extracts, one or more eugenol-bearing vegetable oils and optionally usual stabilising agents and additives,
**characterised by**
(a) 40 to 60 parts by weight of the medicinal herb extracts of aloe, myrrh, safflower, senna leaves, manna, carline thistle, angelica root, camphor, zedoary root and theriak, and
(b) 60 to 40 parts by weight of the vegetable oil clove oil, wherein
(c) the medicinal herb extracts are present in substantially equal amounts with each other.

2. A food supplement according to claim 1 **characterised in that** in addition the medicinal herb extracts of common nettle and mistletoe are present in substantially the same amount as the other medicinal herb extracts.

3. A food supplement according to claim 1 or claim 2 **characterised in that** in addition the medicinal herb extracts of marigold, galium, celandine, mallow and mistletoe are present in substantially the same amount as the other medicinal herb extracts and in addition the vegetable oil laurel leaf oil is present, wherein the amount of the additional vegetable oil is 10 to 12% by weight of the amount of the clove oil.

4. A food supplement according to claim 1 **characterised in that** in addition the medicinal herb extracts of galium, calamus, celandine, willow herb, mistletoe and field horsetail are present in substantially the same amount as the other medicinal herb extracts and in addition the vegetable oils laurel leaf oil, eucalyptus oil and citronella oil are present, wherein the amount of the additional vegetable oils is in each case 10 to 12% by weight of the amount of the clove oil.

5. A food supplement according to claim 1 **characterised in that** in addition the medicinal herb extracts of common nettle, marigold, calamus, celandine, mallow and yarrow are present in substantially the same amount as the other medicinal herb extracts, and in addition the vegetable oils bay oil, laurel leaf oil, basil oil and citronella oil are present, wherein the amount of the additional vegetable oils is in each case 10 to 12% by weight of the amount of the clove oil.

6. A food supplement according to claim 1 **characterised in that** in addition the medicinal herb extracts of common nettle, marigold, galium, wood sorrel, calamus, celandine, mallow, willow herb, yarrow, mistletoe, field horsetail, rosemary, sage and marjoram are present in substantially the same amount as the other medicinal herb extracts and in addition the vegetable oils pimento berry oil, bay oil, laurel leaf oil, basil oil, eucalyptus oil and citronella oil are present, wherein the amount of the additional vegetable oils is in each case 10 to 12% by weight of the amount of the clove oil.

7. A food supplement according to one of the preceding claims **characterised in that** in relation to the sum of medicinal herb contents, vegetable oils and ethanol, it contains 8 to 12% by weight of ethanol and 92 to 88% by weight of medicinal herb contents plus vegetable oils.

8. A food supplement according to one of the preceding claims **characterised in that** it includes an emulsifier.

9. A food supplement according to claim 8 **characterised in that** the emulsifier is lecithin.

10. A food supplement according to one of claims 1 to 9 **characterised in that** aloe is increased in relation to the amount in accordance with feature (c) and at the same time safflower is reduced in such a fashion that the increase and the reduction compensate each other.

11. A food supplement according to one of claims 2 to 10 **characterised by** 45 to 55 parts by weight of medicinal herb extracts to 55 to 45 parts by weight of vegetable oil or oils.

## Revendications

1. Complément nutritionnel renforçant l'immunité, contenant un ou plusieurs extraits éthanoliques de plantes médicinales, une ou plusieurs huiles essentielles végétales contenant de l'eugénol ainsi que, le cas échéant, des agents stabilisants et des additifs usuels, **caractérisé en ce qu'**il contient :
(a) de 40 à 60 parties en poids d'extraits des plantes médicinales aloès, myrrhe, carthame, feuilles de séné, manne, scrofulaire, racine d'angélique, camphre, racine de salsepareille et thériaque, et
(b) de 60 à 40 parties en poids d'huile essentielle de girofle,
(c) les extraits de plantes médicinales étant présents en quantités sensiblement égales.

2. Complément nutritionnel selon la revendication 1, **caractérisé en ce qu'**il contient en plus des extraits des plantes médicinales ortie et gui en quantité sensiblement identique à celle des autres extraits de plantes médicinales.

3. Complément nutritionnel selon la revendication 1 ou la revendication 2, **caractérisé en ce qu'**il contient en plus des extraits des plantes médicinales souci, gaillet, chélidoine, mauve des chemins et gui en quantité sensiblement identique à celle des autres extraits de plantes médicinales et **en ce qu'**il contient en plus de l'huile essentielle de feuilles de laurier, la quantité de l'huile essentielle supplémentaire étant de 10 à 12% en poids ramenée à la quantité d'huile essentielle de girofle.

4. Complément nutritionnel selon la revendication 1, **caractérisé en ce qu'**il contient en plus des extraits des plantes médicinales gaillet, acore, chélidoine, épilobe, gui et prêle des champs qui sont présents en quantité sensiblement identique à celle des autres extraits de plantes médicinales et **en ce qu'**il contient en plus les huiles essentielles végétales de feuilles de laurier, d'eucalyptus et de citronnelle, la quantité respective des huiles essentielles végétales supplémentaires étant de 10 à 12% en poids ramenée à la quantité d'huile essentielle de girofle.

5. Complément nutritionnel selon la revendication 1, **caractérisé en ce qu'**il contient en plus les extraits de plantes médicinales ortie, souci, acore, chélidoine, mauve des chemins et gaillet en une quantité sensiblement identique à celle des autres extraits de plantes médicinales et **en ce qu'**il contient en plus les huiles essentielles végétales d'oléandre, de feuilles de laurier, de basilic et de citronnelle, la quantité respective des huiles essentielles végétales supplémentaires étant de 10 à 12% en poids ramenée à la quantité d'huile essentielle de girofle.

6. Complément nutritionnel selon la revendication 1, **caractérisé en ce qu'**il contient en plus les extraits de plantes médicinales ortie, souci, oseille, acore, chélidoine, mauve des chemins, épilobe, gaillet, gui, prêle des champs, romarin, sauge et marjolaine et **en ce qu'**il contient en plus les huiles essentielles végétales de baies de piment, d'oléandre, de feuilles de laurier, de basilic, d'eucalyptus et de citronnelle, la quantité respective des huiles essentielles végétales supplémentaires étant de 10 à 12% en poids ramenée à la quantité d'huile essentielle de girofle.

7. Complément nutritionnel selon la revendication 1, **caractérisé en ce qu'**il contient de 8 à 12% en poids d'éthanol et de 92 à 88% en poids d'ingrédients extraits de plantes médicinales et huiles essentielles végétales, ramené à la somme des ingrédients extraits de plantes médicinales, des huiles essentielles végétales et de l'éthanol.

8. Complément nutritionnel selon l'une des revendications précédentes, **caractérisé en ce qu'**il contient un émulsifiant.

9. Complément nutritionnel selon la revendication 8, **caractérisé en ce que** l'émulsifiant est la lécithine.

10. Complément nutritionnel selon l'une des revendications 1 à 9, **caractérisé en ce que** la quantité d'aloès est augmenté par rapport à la caractéristique (c) et **en ce que**, dans le même temps, la quantité de carthame est réduite de façon que l'augmentation et la diminution se compensent.

11. Complément nutritionnel selon l'une des revendications 2 à 10, **caractérisé en ce qu'**il contient de 45 à 55% parties en poids d'extraits de plantes médicinales pour 55 à 45 parties en poids d'huile(s) essentielle (s) végétale(s).
